# EUROPÄISCHE PATENTSCHRIFT

(11) **EP 3 942 941 B1**
(45) Veröffentlichungstag und Bekanntmachung des Hinweises auf die Patenterteilung: **04.12.2024**
(21) Anmeldenummer: 21187309.6
(22) Anmeldetag: 22.07.2021
(51) Int. Cl.: A61L 2/03, A23L 3/32

(54) **KOMPAKTANLAGE FÜR GEPULSTE ELEKTRISCHE FELDER IN BEHANDLUNGSZELLE**
COMPACT SYSTEM FOR PULSED ELECTRIC FIELDS IN A TREATMENT CELL
INSTALLATION COMPACTE POUR CHAMPS ÉLECTRIQUES PULSÉS DANS UNE CELLULE DE TRAITEMENT

(30) Priorität: 23.07.2020 DE 102020209334
(43) Veröffentlichungstag der Anmeldung: 26.01.2022
(73) Patentinhaber: Deutsches Institut für Lebensmitteltechnik e.V., 49610 Quakenbrück (DE)
(72) Erfinder: Kleemann, Tobias, 49610 Quakenbrück (DE); Isaak, Johann, 49610 Quakenbrück (DE); Stute, Martin, 49610 Quakenbrück (DE)
(74) Vertreter: Taruttis, Stefan Georg

(56) Entgegenhaltungen:
- US-A- 5 549 041
- US-A1- 2008 063 579
- US-A1- 2020 008 449
- TAGHAVI-MOGHADAM SHAHRIYAR ET AL: "Microreaction Technology as a Novel Approach to Drug Design, Process Development and Reliability", ORGANIC PROCESS RESEARCH & DEVELOPMENT, vol. 5, no. 6, 16 November 2001 (2001-11-16), US, pages 652 - 658, XP055834469, ISSN: 1083-6160, DOI: 10.1021/op010066u
- HUANG K ET AL: "Designs of pulsed electric fields treatment chambers for liquid foods pasteurization process: A review", JOURNAL OF FOOD ENGINEERING, ELSEVIER, AMSTERDAM, NL, vol. 95, no. 2, 1 November 2009 (2009-11-01), pages 227 - 239, XP026419536, ISSN: 0260-8774, [retrieved on 20090614]

## Beschreibung

Die vorliegende Erfindung betrifft eine Kompaktanlage zur Erzeugung gepulster elektrischer Felder zwischen Elektroden in einer Behandlungszelle, um darin ein Medium, flüssig oder fest, bevorzugt wässrig, mit gepulsten elektrischen Feldern zu beaufschlagen.

Die Kompaktanlage hat den Vorteil, dass ein einfaches Wechseln der Behandlungszelle, die Elektroden enthält, möglich ist und beim Wechseln auch elektrische Leitungen, die elektrische Impulse leiten, betriebssicher mit den Elektroden verbunden werden.

### Stand der Technik

Die DE 10 2009 034 707 B4 beschreibt zur Behandlung flüssiger Medien mit gepulsten elektrischen Feldern zwei in einem Abstand voneinander angeordnete Elektroden, die in einem elektrisch nichtleitenden Gehäuse angeordnet sind, wobei eine der Elektroden eine Leitung zur Zuführung des Mediums zwischen die Elektroden bildet.

Huang und Wang, Journal of Food Engineering 95 (2009) 227-239 zeigt in einem Gehäuse eine Durchflusszelle, die mit elektrischer Spannung beaufschlagt werden kann.

Die WO 2018/138361 A1 beschreibt ein Förderband mit zwei seitlich über dem Förderband stehenden Elektroden, die mittels massiver Stromschienen mit einer Spannungsversorgung verbunden sein können.

Die US 5,549,041 beschreibt eine PEF-Vorrichtung mit einem am Rahmen zu befestigenden Behälter, dessen Wände Elektroden bilden, die durch Federkraft gegen Querschnittsöffnungen einer Kammer gepresst werden.

Maghadam et al., Organic Process R & D, 2001 beschreibt ein Mikroreaktorsystem für chemische Synthesen, bei dem eine Reaktionskammer in einen Plattenstapel eingebettet ist, der tauschbar in eine verschließbare Kammer eingesetzt werden kann.

Die US 2008/0063579 A1 beschreibt eine Vorrichtung zum Frischhalten von Lebensmitteln durch Anlegen eines elektrischen Feldes an das Lebensmittel, wobei das elektrische Feld durch flexible Elektroden erzeugt wird.

Die US 2004/0037736 A1 beschreibt Vorrichtung und Verfahren zum Sterilisieren von Medizinprodukten in einer Kammer durch doppelte Plasmaentladung.

### Aufgabe der Erfindung

Der Erfindung stellt sich die Aufgabe, eine kompakte Anlage zur Behandlung von Medien mit gepulsten elektrischen Feldern anzugeben, die das Wechseln der Behandlungszelle, die Elektroden aufweist, bei einfacher Handhabung erlaubt. Bei der Anlage soll bevorzugt die elektrische Verbindung der Elektroden mit einem Generator, der elektrische Impulse erzeugen kann, hergestellt werden können, ohne dass ein Elektrofachmann zur Montage elektrischer Leitungen erforderlich ist.

### Beschreibung der Erfindung

Die Erfindung löst die Aufgabe mit den Merkmalen der Ansprüche und insbesondere mit einer Vorrichtung, die eine Kompaktanlage bildet. Die Vorrichtung weist in einem Gehäuse eine von einer Klappe verschließbare Kammer auf, von der eine Wand zumindest ein erstes Führungselement, bevorzugt zwei erste Führungselemente, und zumindest ein erstes Fixierelement, sowie zwei voneinander beabstandete erste Kontaktelemente aufweist, die mit elektrischen Leitungen fest mit elektrischen Anschlüssen eines Generators verbunden sind.

Weiter weist die Vorrichtung zumindest einen Träger, bevorzugt zumindest zwei Träger auf, der z.B. plattenförmig sein kann und auch als Trägerplatte bezeichnet wird. Der Träger weist zumindest ein zweites Führungselement, bevorzugt zwei zweite Führungselemente auf, die bei Anordnung des Trägers gegen die Wand mit den ersten Führungselementen in Eingriff treten und den Träger in eine vorbestimmte Stellung zur Wand ausrichten. Der Träger weist zumindest ein zweites Fixierelement auf, das bei Eingriff der ersten und zweiten Führungselemente passend zu dem zumindest einen ersten Fixierelement ausgerichtet ist, so dass erste und zweite Fixierelemente bei Eingriff der ersten und zweiten Führungselemente passend zueinander ausgerichtet sind und aneinander befestigt werden können.

Optional können die ersten Führungselemente und ersten Fixierelemente von jeweils einem Bauteil gebildet werden, und die zweiten Führungselemente und zweiten Fixierelemente entsprechend von jeweils einem gemeinsamen Bauteil gebildet werden. Bevorzugt sind die ersten Führungselemente und die ersten Fixierelemente von jeweils einzelnen Bauteilen gebildet, die an der Wand festgelegt sind, und die zweiten Führungselemente und die zweiten Fixierelemente sind von jeweils einzelnen Bauelementen gebildet, die am Träger angeordnet sind, festgelegt oder beweglich.

Bevorzugt ragen die ersten Führungselemente über die Wand hinaus und die zweiten Führungselemente sind Ausnehmungen an oder in dem Träger, die über die ersten Führungselemente beweglich sind. Z.B. können die ersten Führungselemente Bolzen sein, die über die Wand ragen und die zweiten Führungselemente können umfänglich geschlossene oder zum Rand des Trägers offene Ausnehmungen im Träger sein, die über die Bolzen gehoben und/oder geschoben werden können.

Die ersten Fixierelemente können in der Wand angebrachte Innengewinde sein und die zweiten Fixierelemente Schrauben, die drehbar an dem Träger geführt sind.

In einer Ausführungsform, in der die ersten Führungselemente und ersten Fixierelemente von einem Bauteil gebildet werden, kann ein solches erstes Bauteil von einem Gewindebolzen gebildet sein, der die Wand überragt, und die zweiten Führungselemente können von jeweils passend zu den Gewindebolzen in dem Träger gebildeten Ausnehmungen sein und die zweiten Fixierelemente können Muttern sein, die über die Gewindebolzen geschraubt werden können.

Die in der Wand angeordneten ersten Führungselemente und ersten Fixierelemente und die dazu in oder an dem Träger passend angeordneten zweiten Führungselemente und zweiten Fixierelemente haben den Vorteil, dass ein Träger einfach und zu einer vorbestimmten Anordnung passend an der Wand angeordnet und befestigt werden kann.

Der Träger weist zumindest zwei zweite Kontaktelemente auf, die passend zu den ersten Kontaktelementen angeordnet sind, wenn die Führungselemente in Eingriff sind und der Träger gegen die Wand angeordnet wird. Die ersten Kontaktelemente erstrecken sich bevorzugt ausschließlich hinter der der Kammer zugewandten ersten Oberfläche der Wand, bzw. die ersten Kontaktelemente sind bevorzugt ausschließlich hinter der der Kammer zugewandten ersten Oberfläche der Wand angeordnet, so dass die ersten Kontaktelemente bevorzugt nicht über die der Kammer zugewandte erste Oberfläche der Wand ragen.

Die zweiten Kontaktelemente sind am Träger in Stellungen festgelegt, die passend zu den ersten Kontaktelementen ausgerichtet sind und mit diesen in Eingriff treten, wenn der Träger bei Eingriff der ersten und zweiten Führungselemente miteinander an der Wand angeordnet wird. Die zweiten Kontaktelemente ragen über die zweite Oberfläche des Trägers, die bei Anordnung des Trägers an der Wand dieser zugewandt ist.

An der ersten Oberfläche des Trägers ist eine Behandlungszelle für Medium angebracht, die zumindest zwei Elektroden, isoliert oder elektrisch leitend, aufweist, die jeweils einen Teil der Behandlungszelle bilden, um zwischen sich elektrische Impulse an das Medium abzugeben, bzw. zwischen sich gepulste elektrische Felder in dem Medium zu erzeugen. Die Elektroden sind durch Leitungen mit den zweiten Kontaktelementen verbunden. Bevorzugt besteht der Träger zumindest in dem Bereich, in dem die Leitungen zwischen den zweiten Kontaktelementen und den Elektroden angeordnet sind und in dem Bereich, in dem die Elektroden der Behandlungszelle angeordnet sind, aus Isoliermaterial, z.B. nichtleitendem Kunststoff. Weiter bevorzugt besteht der Träger vollständig aus Isoliermaterial.

Weiter bevorzugt besteht die Wand, bevorzugter alle Wände, die die Kammer begrenzen, aus Isoliermaterial. Bevorzugt besteht die Klappe aus transparentem Isoliermaterial, z.B. Glas oder Kunststoff, z.B. Polycarbonat oder Polyacrylat. Die Klappe kann schenkbar oder verschieblich am Gehäuse geführt sein und sie kann einteilig oder mehrteilig sein.

Bei Anordnung der ersten und zweiten Führungselemente aneinander werden dadurch elektrische Leitungen von den Anschlüssen des Generators zu den Elektroden hergestellt, dass die zweiten Kontaktelemente passend zu den ersten Kontaktelementen ausgerichtet sind und in diese eingreifen. Da die elektrischen Leitungen von den Elektroden zu zweiten Kontaktelementen festgelegt sind und die elektrischen Leitungen von den ersten Kontaktelementen zu Anschlüssen des Generators festgelegt sind, ist die Vorrichtung eingerichtet, die Elektroden der Behandlungszelle allein durch die Verbindung bzw. das Eingreifen der ersten und zweiten Kontaktelemente mit dem Generator elektrisch zu verbinden. Dazu ist es nicht erforderlich, an den Leitungen selbst zu manipulieren. Daher bilden die ersten Kontaktelemente mit den zweiten Kontaktelementen die einzige trennbare Verbindung in den elektrischen Leitungen zwischen den Elektroden und dem Generator, während elektrische Leitungen fest die Elektroden und den zweiten Kontaktelementen verbinden und elektrische Leitungen fest die ersten Kontaktelemente mit den Anschlüssen des Generators verbinden. Diese festen Verbindungen können dadurch hergestellt sein, dass die Leitungen unlösbar an den Elektroden und den zweiten Kontaktelementen verbunden sind, z.B. mittels abgescherter Schrauben oder durch Schweiß- oder Lötverbindungen, an einem Ende mit den Elektroden und am anderen Ende mit den zweiten Kontaktelementen verbunden sind. Optional sind auch die Leitungen zwischen den ersten Kontaktelementen und dem Generator unlösbar an einem Ende mit den ersten Kontaktelementen und am anderen Ende mit den Anschlüssen des Generators verbunden. Der Generator ist bevorzugt ein Hochspannungsimpulsgenerator. Die verbundenen Leitungen können z.B. fest verdrahtet sein.

Die erfindungsgemäße Anordnung der ersten Kontaktelemente hinter der ersten Oberfläche der Wand, die der Kammer zugewandt ist, verhindert ein unbeabsichtigtes Kontaktieren der ersten Kontaktelemente.

Optional können zwei oder mehr Elektroden gleicher Polarität an oder in der Behandlungszelle angebracht sein, und die Elektroden gleicher Polarität können mit demselben zweiten Kontaktelement mittels einer gemeinsamen Leitung oder mehrerer einzelner Leitungen verbunden sein.

Bevorzugt liegt die Vorrichtung in Kombination mit zumindest einem zusätzlichen Träger vor, bzw. die Vorrichtung weist zumindest zwei Träger auf, deren zweite Fixierelemente, zweite Führungselemente und zweite Kontaktelemente dieselbe Anordnung aufweisen. Die zumindest zwei Träger können mittels der Führungselemente und Fixierelemente abwechselnd an der Wand angeordnet werden, wobei die Führungselemente die zweiten Kontaktelemente jeweils passend zu den ersten Kontaktelementen ausrichten. Entsprechend liegt die Vorrichtung bevorzugt in Kombination mit zumindest zwei Trägern vor, deren zweite Fixierelemente, zweite Führungselemente und zweite Kontaktelemente jeweils dieselbe Anordnung aufweisen, so dass sie passend zu den ersten Fixierelementen, ersten Führungselementen und ersten Kontaktelementen angeordnet sind.

Bei einem Träger, bevorzugt bei zumindest zwei Trägern, kann jeder Träger z.B. eine in sich geschlossene Behandlungszelle für eine satzweise elektrische Beaufschlagung eines in der Behandlungszelle enthaltenen Mediums aufweisen, oder eine Behandlungszelle mit einem Einlass und einem Auslass für Medium für eine elektrische Beaufschlagung des Mediums während die Behandlungszelle vom Medium durchströmt wird.

Optional weist die Klappe Ausnehmungen auf, durch die zumindest eine erste Leitung für die Zuführung von Medium in die Behandlungszelle, bevorzugt zumindest eine zweite Leitung für das Abführen von Medium aus der Behandlungszelle angeordnet werden kann.

Bevorzugt ist zwischen der Klappe und dem Gehäuse ein Sicherheitsschalter angebracht, der eingerichtet ist, den Generator nur bei über der Kammer geschlossener Klappe mit einem Stromanschluss zu verbinden, und/oder den Generator bei Offenstellung der Klappe von einem Stromanschluss zu trennen.

Ein Generator kann in einem separaten Generatorgehäuse angeordnet sein, das mit dem Gehäuse verbunden ist, z.B. an aneinander anliegenden äußeren Gehäusewänden. Bevorzugt ist der Generator innerhalb des einen Gehäuses angeordnet, in dem auch die Kammer angeordnet ist. Diese Ausführungsform hat den Vorteil, dass der Generator und die elektrischen Leitungen, die die Anschlüsse des Generators mit den Elektroden verbinden können, vollständig innerhalb eines gemeinsamen Gehäuses verlaufen. Bevorzugt weist das Gehäuse eine Steuerung für den Generator auf, z.B. zur Einstellung von Parametern der elektrischen Impulse.

Die elektrischen Leitungen von den Anschlüssen des Generators zu den ersten Kontaktelementen und/oder die elektrischen Leitungen von den zweiten Kontaktelementen zu den Elektroden der Behandlungszelle können einadrige Leitungen oder massive Leiter sein, jeweils optional mit einer äußeren Schirmung.

Weiter bevorzugt weist die Behandlungszelle Sensoren auf, die direkt oder optional durch Leitungen für Medium mit der Behandlungszelle verbunden sein können. Bevorzugt umfassen die Sensoren zumindest einen Temperatursensor, zumindest einen Drucksensor und/oder zumindest einen Leitfähigkeitssensor, die jeweils in Kontakt mit dem Innenraum der Behandlungszelle stehen. Dabei weist das Gehäuse eine Messelektronik auf, die zum Empfang und Auswertung von Sensorsignalen eingerichtet ist. Die Messelektronik ist in dem Gehäuse enthalten und bevorzugt mit einer Anzeige, die am Gehäuse zugänglich ist. Die Anzeige kann in Form einer Sendeeinheit gebildet sein, die eingerichtet ist, Sensorsignale aus dem Gehäuse zu senden, z.B. in Form von Funksignalen. Solche z.B. in Form von Funksignalen gesendeten Sensorsignale können z.B. mit einem dafür eingerichteten Empfänger empfangen werden, der vom Gehäuse entfernt sein kann.

Weiter optional ist die Messelektronik mit der Steuerung für den Generator verbunden und die Steuerung ist eingerichtet, den Generator in Abhängigkeit von Signalen der Messelektronik zu regeln.

Bevorzugt weist jeder Träger auf seiner ersten Oberfläche oder auf seiner zweiten Oberfläche eine optische Kennung auf, z.B. einen Strichkode, und ein optischer Detektor ist an oder in der Kammer, z.B. an oder in der Wand, gegen die der Träger angeordnet wird, in einer Stellung angeordnet, gegenüber der die optische Kennung bei Eingriff der Führungselemente von Träger und Wand, optional bei Fixierung der ersten und zweiten Fixierelemente aneinander, angeordnet ist. Der optische Detektor ist bevorzugt zur Übermittlung von Signalen, die auf Basis der optischen Kennung vom Detektor aufgenommen werden, an den Generator eingerichtet. In dieser Ausführungsform ist bevorzugt, dass die optische Kennung spezifisch für die Elektroden und/oder für die Art der Behandlungszelle, z.B. geschlossene Behandlungszelle oder mit Zuführleitung und/oder Abführleitung für Medium aus der Behandlungszelle ist, so dass das vom Detektor aufgenommene Signal als Identifizierung der Behandlungszelle dient. Weiter optional ist die Vorrichtung eingerichtet, das Signal als Identifizierung des Trägers an den Generator zu leiten, und die Vorrichtung ist eingerichtet, den Generator abhängig vom Signal zu steuern, z.B. maximale Werte für Parameter der elektrischen Impulse vorzugeben.

Es hat sich gezeigt, dass die optische Kennung an dem Träger und ein optischer Detektor, z.B. an oder in der Wand der Kammer den Vorteil hat, dass die Identifizierung des Trägers ohne Beeinträchtigung durch elektrische Felder und daher betriebssicher erfolgen kann. Ein optischer Detektor kann eine Anordnung von Photosensoren oder eine optische Kamera sein.

Die Erfindung wird nun genauer mit Bezug auf die Figuren beschrieben, die in
- Fig. 1 eine Gesamtansicht einer Ausführungsform der Vorrichtung,
- Fig. 2 abschnittsweise eine Ausführungsform der Vorrichtung in Aufsicht,
- Fig. 3 die Ausführungsform von Fig. 1 in seitlicher Schnittansicht,
- Fig. 4 abschnittsweise eine weitere Ausführungsform der Vorrichtung in Aufsicht,
- Fig. 5 die Ausführungsform von Fig. 3 in seitlicher Schnittansicht und in
- Fig. 6 eine Ausführungsform der ersten und zweiten Kontaktelemente
zeigen.

In den Figuren bezeichnen gleiche Bezugsziffern funktionsgleiche Elemente.

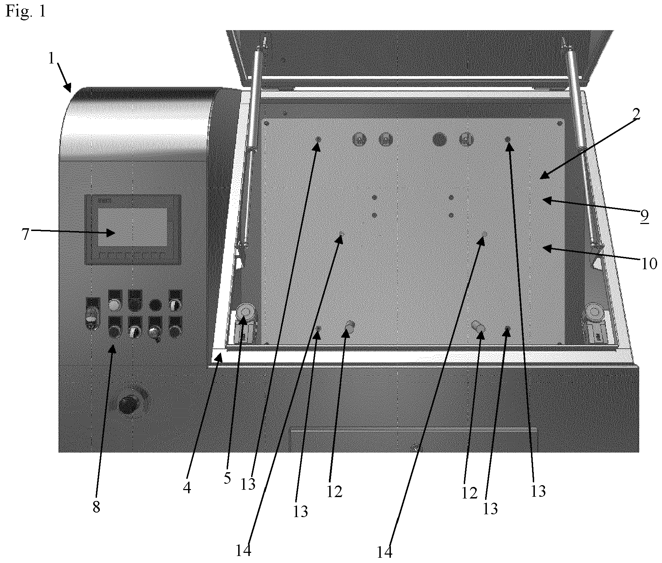

Die Fig. 1 zeigt ein gemäß der bevorzugten Ausführungsform ein Gehäuse 1, in dem sowohl ein Generator für die Erzeugung von elektrischen Impulsen integriert ist, als auch eine Kammer 2, die von einer am Gehäuse 1 schwenkbar angelenkten Klappe 3 verschließbar ist. Am Gehäuse 1 ist im Bereich des Rahmens 4, auf dem die Klappe 3 in der Schließposition aufliegt, ein Sicherheitsschalter 5 angebracht, der von seinem Gegenstück 6 an der Klappe 3 geschaltet wird und bei Schließposition der Klappe 3 ein Signal erzeugt, das die Verbindung des Generators mit seinem Stromanschluss zulässt und das bei Offenstellung der Klappe 3 die Verbindung des Generators zu seinem Stromanschluss trennt. Das Gehäuse 1 weist in der hier gezeigten Ausführungsform eine Anzeige 7 auf, die z.B. Parameter der vom Generator erzeugten elektrischen Impulse und optional von Sensoren aufgenommene Parameter zeigt, sowie Betätigungsschalter 8 für den Generator. Die rückseitige Wand 10 der Kammer 2 ist zur Aufnahme eines Trägers 20 vorbereitet und weist zwei erste Führungselemente 12 in Form von Bolzen auf, die über die Wand 10 ragen. In der Wand 10 sind separat von den ersten Führungselementen erste Fixierelemente 13 in Form von Gewindebohrungen angebracht. Weiter sind in der Wand 10 zwei erste Kontaktelemente 14 angeordnet, die in der Wand versenkt sind und sich nur hinter der der Kammer 2 zugewandten ersten Oberfläche 9 der Wand 10 in das Gehäuse 1 erstrecken.

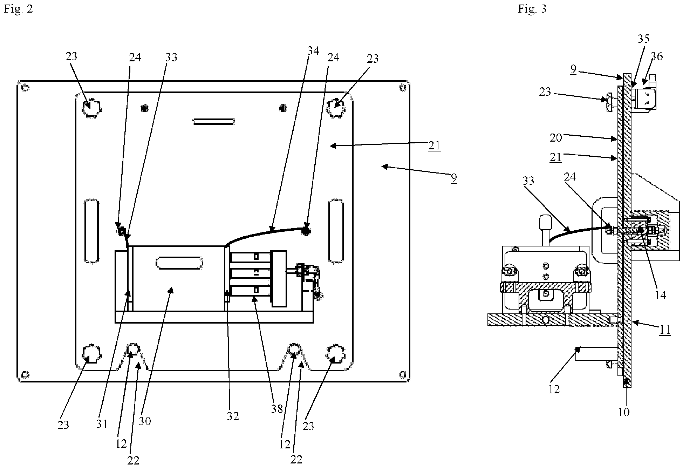

Die Fig. 2 zeigt in Aufsicht auf die Wand 10 einen daran angeordneten Träger 20, dessen erste Oberfläche 21 der Wand 10 gegenüberliegt. Am Träger 20 sind zweite Führungselemente 22 in Form von Ausnehmungen angeordnet, die entlang der ersten Führungselement 12 verschieblich sind und darauf positioniert sind. Die am Träger 20 angeordneten zweiten Fixierelemente 23 sind bei Eingriff der ersten und zweiten Führungselemente 12, 22 passend zu den ersten Fixierelementen 13 angeordnet. Am Träger 20 sind zwei zweite Kontaktelemente 24 angeordnet, die über die zweite Oberfläche 25 des Trägers 20 ragen, die der Wand 10 zugewandt ist, bei Eingriff der ersten und zweiten Führungselemente 12, 22 passend zu den ersten Kontaktelementen 14 ausgerichtet sind und in diese eingreifen. An der ersten Oberfläche 21 des Trägers 20 ist eine Behandlungszelle 30 angebracht, die zumindest zwei Elektroden 31, 32 aufweist. Die Elektroden 31, 32 sind mit jeweils separaten elektrischen Leitungen 33, 34 mit einem der zweite Kontaktelemente 24 verbunden.

Fig. 3 zeigt den Ausschnitt von Fig. 2 um 90° gedreht im Schnitt. Auf der zweiten Oberfläche 11 der Wand 10, gegenüber ihrer ersten Oberfläche 9, ist gemäß einer Ausführungsform eine optische Kennung 35 für den Träger bzw. für die darauf angebrachte Behandlungszelle 30 angebracht, und ein optischer Detektor 36 liegt bei Anordnung des Trägers 20 gegen die Wand 10 über der optischen Kennung 35. Der Detektor 36 ist eingerichtet, ein Signal für die optische Kennung 35 an den Generator 37 zu übermitteln, um den Generator 37 abhängig von diesem Signal zu steuern.

Die in Fig. 2 und Fig. 3 gezeigte beispielhafte Behandlungszelle 30 ist in einem Spannrahmen 38 angeordnet, mit dem die Elektroden 31, 32 aufeinander zu gespannt werden könne, so dass die Elektroden 31, 32 abhängig von der Behandlungszelle 30 in verschiedenen Abständen angeordnet sein können. Diese Behandlungszelle 30 kann ohne angeschlossene Leitung für Medium zur satzweisen Behandlung eines Mediums verwendet werden.

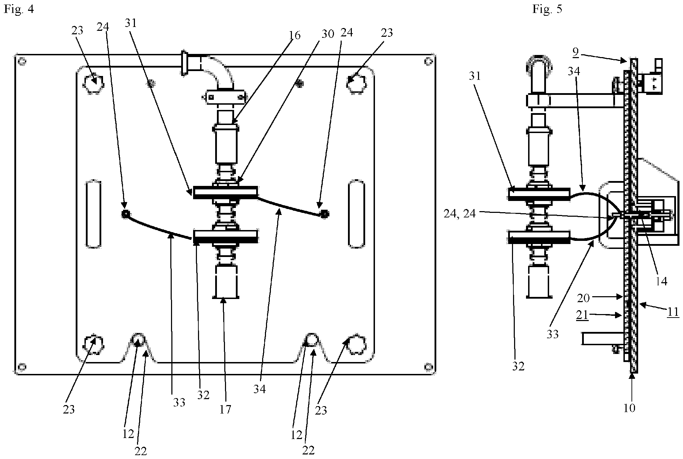

Die Fig. 4 zeigt eine weitere Ausführungsform in Aufsicht auf die Wand 10 einer Kammer 2, und die Fig. 5 zeigt diese Ausführungsform im Schnitt, um 90° gedreht. Die Wand 10 ist dieselbe wie die in Fig. 2 und Fig. 3 gezeigte Wand 10, mit den daran angeordneten ersten Führungselementen 12 und ersten Fixierelementen 13, sowie ersten Kontaktelementen 14. Der hier dargestellte Träger 20 weist an denselben Positionen wie der Träger 20 von Fig. 2 und Fig. 3 die zweiten Führungselemente 22, die zweiten Fixierelement 23 und die zweiten Kontaktelemente 24 auf. Daher können die Träger 20 abwechselnd mit ihren zweiten Führungselementen 22 entlang der ersten Führungselemente 12 an der Wand 10 angeordnet werden, dort mittels ihrer zweiten Fixierelemente 23 fixiert werden, wobei die zweiten Kontaktelemente 24 der Träger 20 jeweils in dieselben ersten Kontaktelemente 14 der Wand 10 eingreifen und die Elektroden 31, 32 mit dem Generator 37 elektrisch verbinden.

In der Ausführungsform von Fig. 4 ist die Behandlungszelle zur Erzeugung von gepulsten elektrischen Feldern geeignet, während Medium entlang der Elektroden strömt. Dazu weist die Behandlungszelle eine erste Leitung 16 für die Zuführung von Medium in die Behandlungszelle 30 und bevorzugt zumindest eine zweite Leitung 17 für das Abführen von Medium aus der Behandlungszelle auf.

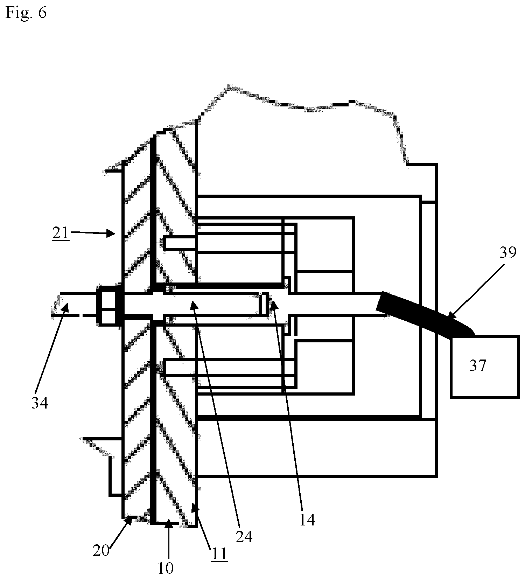

Die Fig. 6 zeigt eine bevorzugte Ausführungsform eines ersten Kontaktelements 14 im Eingriff mit einem zweiten Kontaktelement 24 im Schnitt. Dabei erstreckt sich das erste Kontaktelement 14 nur in und/oder hinter der Wand 10 von deren zweiter Oberfläche 11, die ihrer ersten Oberfläche 9 und dem daran angeordneten Träger 20 gegenüberliegt. Das zweite Kontaktelement 14 weist eine Ausnehmung 15 auf, die elektrisch leitfähig ist, z.B. eine Bohrung in Metall. Das zweite Kontaktelement 24 ragt über den Träger 20 und in die Ausnehmung 14 des ersten Kontaktelements 14. Jedes erste Kontaktelement 14 ist mittels einer elektrischen Leitung 39 mit einem Anschluss des Generators 37 fest verbunden.

### Bezugszeichen:

1 Gehäuse
2 Kammer
3 Klappe
4 Rahmen
5 Sicherheitsschalter
6 Gegenstück des Sicherheitsschalters
7 Anzeige
8 Betätigungsschalter
9 erste Oberfläche der Wand
10 Wand
11 zweite Oberfläche der Wand
12 erstes Führungselement
13 erstes Fixierelement
14 erstes Kontaktelement
15 Ausnehmung
16 erste Leitung für Medium
17 zweite Leitung für Medium
20 Träger
21 erste Oberfläche des Trägers
22 zweite Führungselemente am Träger
23 zweite Fixierelemente am Träger
24 zweites Kontaktelement
25 zweite Oberfläche des Trägers
15 Ausnehmung des ersten Kontaktelements
30 Behandlungszelle
31, 32 Elektrode
33, 34 elektrische Leitung
35 optische Kennung
36 optischer Detektor
37 Generator
38 Spannrahmen
39 elektrische Leitung

## Patentansprüche

1. Vorrichtung zur Behandlung eines Mediums mit gepulsten elektrischen Feldern, mit einer Behandlungszelle (30), die zumindest zwei Elektroden (31, 32) aufweist, die mittels elektrischer Leitungen (33, 34, 39) mit Anschlüssen eines Generators (37) für elektrische Impulse verbunden sind, wobei die Behandlungszelle (30) in einer Kammer (2) in einem Gehäuse (1) angeordnet ist, die von einer Klappe (3) verschließbar ist, **dadurch gekennzeichnet, dass** die Behandlungszelle (30) an einem Träger (20) angebracht ist und die Kammer (2) eine Wand (10) aufweist, an der zumindest ein erstes Führungselement (12) angeordnet ist, das mit zumindest einem, an dem Träger (20) angeordneten zweiten Führungselement (22) in Eingriff bringbar ist, an der Wand (10) zumindest ein erstes Fixierelement (13) angeordnet ist, an dem bei Eingriff der ersten und zweiten Führungselemente (13, 23) zumindest ein an dem Träger (20) angeordnetes zweites Fixierelement (23) befestigbar ist und dass an der Wand (10) zumindest zwei erste Kontaktelemente (14) angeordnet sind, in die bei Eingriff der ersten und zweiten Führungselemente (13, 23) jeweils ein am Träger (20) angeordnetes zweites Kontaktelement (24) eingreift und die Elektroden (31, 32) mittels elektrischer Leitungen (33, 34) mit den zweiten Kontaktelementen (24) fest verbunden sind und die ersten Kontaktelemente (14) mittels elektrischer Leitungen (39) fest mit den Anschlüssen des Generators (37) verbunden sind.

2. Vorrichtung nach Anspruch 1, **dadurch gekennzeichnet, dass** die ersten Kontaktelemente (14) sich nur innerhalb der Wand (10) und/oder von der dem Träger (20) gegenüberliegenden zweiten Oberfläche (11) der Wand (10) in das Gehäuse (1) erstrecken und die zweiten Kontaktelemente (24) über die der Wand (10) zugewandte zweite Oberfläche (25) des Trägers (20) ragen.

3. Vorrichtung nach einem der voranstehenden Ansprüche, **gekennzeichnet durch** zumindest einen zusätzlichen Träger (20), dessen zweite Fixierelemente (23), zweite Führungselemente (22) und zweite Kontaktelemente (24) jedes Trägers (20) dieselbe Anordnung aufweisen.

4. Vorrichtung nach einem der voranstehenden Ansprüche, **dadurch gekennzeichnet, dass** die Elektroden (31, 32) durch unlösbare elektrische Leitungen (33, 34) mit den zweiten Kontaktelementen (24) verbunden sind.

5. Vorrichtung nach einem der voranstehenden Ansprüche, **dadurch gekennzeichnet, dass** das zumindest eine erste Führungselement (12) über die Wand (10) ragt und das zumindest eine zweite Führungselement (22) von eine Ausnehmung im Träger (20) gebildet wird.

6. Vorrichtung nach einem der voranstehenden Ansprüche, **dadurch gekennzeichnet, dass** der Träger (20) und die Wand (10) jeweils aus Isoliermaterial bestehen.

7. Vorrichtung nach einem der voranstehenden Ansprüche, **dadurch gekennzeichnet, dass** jeweils ein erstes Führungselement (12) und ein erstes Fixierelement (13) von einem gemeinsamen Bauteil gebildet werden.

8. Vorrichtung nach einem der voranstehenden Ansprüche, **dadurch gekennzeichnet, dass** der Generator (37) und die Kammer (2) in einem gemeinsamen Gehäuse (1) angeordnet sind.

9. Vorrichtung nach einem der voranstehenden Ansprüche, **dadurch gekennzeichnet, dass** zwischen der Klappe (3) und dem Gehäuse (1) ein Sicherheitsschalter (5) angebracht ist, der eingerichtet ist, ein Signal zu erzeugen, dass der Generator (37) nur bei über der Kammer (2) geschlossener Klappe (3) mit einem Stromanschluss zu verbinden ist.

## Claims

1. Device for treatment of a medium with pulsed electric fields, having a treatment cell (30) which has at least two electrodes (31, 32) which are connected by means of electrical leads (33, 34, 39) to terminals of a generator (37) for electric pulses, the treatment cell (30) being arranged in a chamber (2) in a housing (1), which can be closed by a flap (3), **characterised in that** the treatment cell (30) is mounted on a carrier (20) and the chamber (2) has a wall (10) on which at least one first guide element (12) is arranged, which can be brought into engagement with at least one second guide element (22) arranged on the carrier (20), at least one first fixing element (13) is arranged on the wall (10), to which at least one second fixing element (23) arranged on the carrier (20) can be fastened upon engagement of the first and second guide elements (13, 23), and **in that** at least two first contact elements (14) are arranged on the wall (10), into which at least one second contact element (24) arranged on the carrier (20) can engage upon engagement of the first and second guide elements (13, 23), and the electrodes (31, 32) are firmly connected to the second contact elements (24) by means of electrical leads (33, 34) and the first contact elements (14) are firmly connected to the terminals of the generator (37) by means of electrical leads (39).

2. Device according to claim 1, **characterised in that** the first contact elements (14) extend into the housing (1) only within the wall (10) and/or from the second surface (11) of the wall (10) opposite the carrier (20), and the second contact elements (24) project beyond the second surface (25) of the carrier (20) facing the wall (10).

3. Device according to one of the preceding claims, **characterised by** at least one additional carrier (20), the second fixing elements (23), second guide elements (22) and second contact elements (24) of each carrier (20) having the same arrangement.

4. Device according to one of the preceding claims, **characterised in that** the electrodes (31, 32) are connected to the second contact elements (24) by non-detachable electrical leads (33, 34).

5. Device according to one of the preceding claims, **characterised in that** the at least one first guide element (12) projects beyond the wall (10) and the at least one second guide element (22) is formed by a recess in the carrier (20).

6. Device according to one of the preceding claims, **characterised in that** the carrier (20) and the wall (10) each consist of insulating material.

7. Device according to one of the preceding claims, **characterised in that** a first guide element (12) and a first fixing element (13) are each formed by a common component.

8. Device according to one of the preceding claims, **characterised in that** the generator (37) and the chamber (2) are arranged in a common housing (1).

9. Device according to one of the preceding claims, **characterised in that** between the flap (3) and the housing (1) a safety switch (5) is fitted, which is set up to generate a signal that the generator (37) is to be connected to a power connection only when the flap (3) is closed over the chamber (2).

## Revendications

1. Dispositif pour le traitement d'un milieu avec des champs électriques pulsés, avec une cellule de traitement (30) qui présente au moins deux électrodes (31, 32) qui sont reliées au moyen de lignes électriques (33, 34, 39) à des raccordements d'un générateur (37) d'impulsions électriques, la cellule de traitement (30) étant disposée dans une chambre (2) dans un boîtier (1), qui peut être fermée par un couvercle (3), **caractérisée en ce que** la cellule de traitement (30) est montée sur un support (20) et la chambre (2) présente une paroi (10) sur laquelle est disposé au moins un premier élément de guidage (12) qui peut être mis en prise avec au moins un deuxième élément de guidage (22) disposé sur le support (20), au moins un premier élément de fixation (13) est disposé sur la paroi (10), auquel peut être fixé au moins un deuxième élément de fixation (23) disposé sur le support (20) lors de l'engagement des premier et deuxième éléments de guidage (13, 23), et **en ce qu'**au moins deux premiers éléments de contact (14) sont disposés sur la paroi (10), dans lesquels au moins un deuxième élément de fixation (23) peut être fixé lors de l'engagement des premier et deuxième éléments de guidage (13, 23) s'engage respectivement un deuxième élément de contact (24) disposé sur le support (20) et les électrodes (31, 32) sont reliées de manière fixe aux deuxièmes éléments de contact (24) au moyen de lignes électriques (33, 34) et les premiers éléments de contact (14) sont reliés de manière fixe aux connexions du générateur (37) au moyen de lignes électriques (39).

2. Dispositif selon la revendication 1, **caractérisé en ce que** les premiers éléments de contact (14) s'étendent dans le boîtier (1) uniquement à l'intérieur de la paroi (10) et/ou à partir de la deuxième surface (11) de la paroi (10) opposée au support (20) et les deuxièmes éléments de contact (24) dépassent de la deuxième surface (25) du support (20) tournée vers la paroi (10).

3. Dispositif selon l'une des revendications précédentes, **caractérisé par** au moins un support supplémentaire (20) dont les deuxièmes éléments de fixation (23), les deuxièmes éléments de guidage (22) et les deuxièmes éléments de contact (24) de chaque support (20) présentent la même disposition.

4. Dispositif selon l'une des revendications précédentes, **caractérisé en ce que** les électrodes (31, 32) sont reliées aux deuxièmes éléments de contact (24) par des lignes électriques indissociables (33, 34).

5. Dispositif selon l'une des revendications précédentes, **caractérisé en ce que** le au moins un premier élément de guidage (12) dépasse de la paroi (10) et le au moins un deuxième élément de guidage (22) est formé par un évidement dans le support (20).

6. Dispositif selon l'une des revendications précédentes, **caractérisé en ce que** le support (20) et la paroi (10) sont chacun en matériau isolant.

7. Dispositif selon l'une des revendications précédentes, **caractérisé en ce que** respectivement un premier élément de guidage (12) et un premier élément de fixation (13) sont formés par un composant commun.

8. Dispositif selon l'une des revendications précédentes, **caractérisé en ce que** le générateur (37) et la chambre (2) sont disposés dans un boîtier commun (1).

9. Dispositif selon l'une des revendications précédentes, **caractérisé en ce qu'**un interrupteur de sécurité (5) est monté entre le couvercle (3) et le boîtier (1), lequel est agencé pour générer un signal indiquant que le générateur (37) ne doit être relié à une prise de courant que lorsque le couvercle (3) est fermé sur la chambre (2).
